# EUROPEAN PATENT APPLICATION

(11) **EP 4 316 455 A1**
(43) Date of publication of application: **07.02.2024**
(21) Application number: 22779433.6
(22) Date of filing: 21.01.2022
(51) Int. Cl.: A61K 6/887, A61K 6/62, A61K 6/76, A61K 6/849, A61K 6/878

(54) **METHOD FOR PRODUCING DENTAL COMPOSITION AND DENTAL COMPOSITION**

(30) Priority: 31.03.2021 JP 2021062369
(71) Applicant: GC Corporation, Sunto-gun, Shizuoka 410-1307 (JP)
(72) Inventor: FUNAYAMA, Naoya, Tokyo 174-8585 (JP); TAKAHASHI, Makoto, Tokyo 174-8585 (JP); FUKUYO, Yuri, Tokyo 174-8585 (JP); MORI, Toshiki, Tokyo 174-8585 (JP); KASAI, Yuki, Tokyo 174-8585 (JP)
(74) Representative: Müller-Boré & Partner Patentanwälte PartG mbB
(86) International application number: PCT/JP2022/002288
(87) International publication number: WO 2022/209203

(57) **Abstract**

A method of producing a dental composition in which a first component has a second component dispersed therein is provided. The first component is a paste including a first polymerizable monomer that is a liquid, the second component includes a solvent in which the first polymerizable monomer is insoluble, and the method includes a step of mixing the first component and the second component.

## Description

### Technical Field

The present invention relates to a method of producing a dental composition and a dental composition.

### Background Art

Polymerizable compositions such as dental composite resins are used in the dental field. The dental composite resins are widely used because they contain a photopolymerization initiator so that they can be polymerized and cured in a timely manner by light irradiation (see, for example, PTL 1 and 2).

It is known that a unique light scattering phenomenon (opalescence) is observed when natural teeth are exposed to light. Dental composite restorative materials aiming at this effect are disclosed (see, for example, PTL 3).

Composite resins with high light scattering have excellent aesthetics with good color blending because they incorporate the color of the natural teeth surrounding the filling part.

### Citation List

### Patent Literature

PTL 1:
   Japanese Patent No. 6793106
PTL 2:
   Japanese Patent No. 6793241
PTL 3:
   Japanese Patent No. 5762405

### Summary of Invention

### Technical Problem

However, in the conventional method using an organic-inorganic composite filler, there is a problem that the operability (fluidity) as a composite resin is lowered because the amount of the filler is increased.

It is an object of the present invention to provide a dental composition capable of obtaining a cured body with high light scattering and having excellent operability.

### Solution to Problem

According to one aspect of the present invention, a method of producing a dental composition in which a first component has a second component dispersed therein is provided. The first component is a paste including a first polymerizable monomer that is a liquid, the second component includes a solvent in which the first polymerizable monomer is insoluble, and the method includes a step of mixing the first component and the second component.

### Advantageous Effects of Invention

According to one aspect of the present invention, a dental composition capable of obtaining a cured body with high light scattering and having excellent operability, can be provided.

### Brief Description of the Drawings

[FIG. 1]

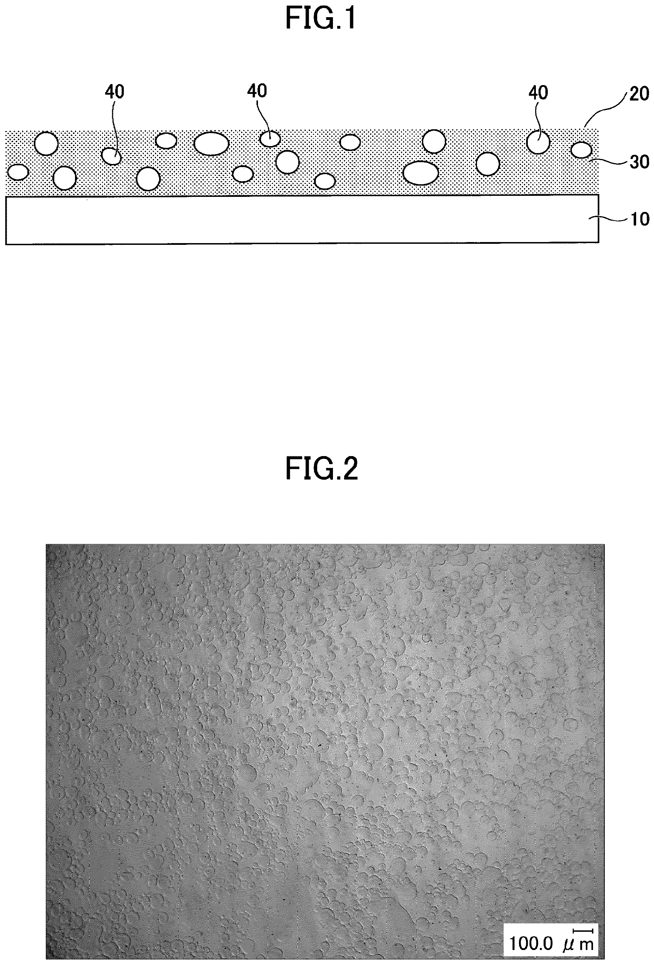

   FIG. 1 is a schematic diagram illustrating a cross section of a dental composition according to an embodiment;
[FIG. 2]
   FIG. 2 is an optical micrograph of the dental composition before curing according to an embodiment; and
[FIG. 3]

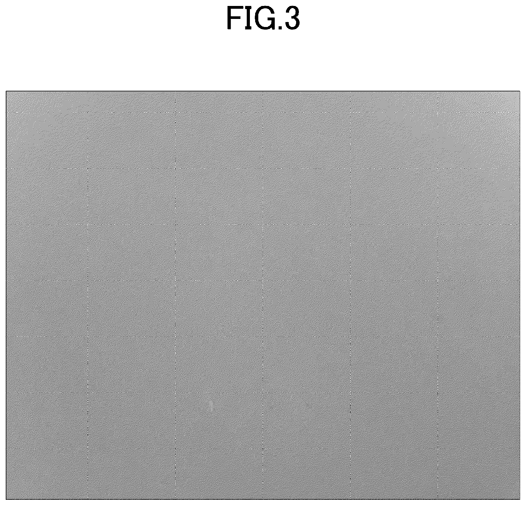

   FIG. 3 is a photograph illustrating an appearance of the dental composition after curing according to an embodiment.

### Description of Embodiments

Hereinafter, embodiments of the present invention will be described in detail.

### <Method of Producing Dental Composition>

A method of producing a dental composition according to the present embodiment is a method of producing a dental composition in which a first component has a second component dispersed therein. The method of producing the dental composition according to the present embodiment includes a step (hereinafter referred to as a mixing step) of mixing the first component and the second component.

### <First Component>

The first component is a paste including a first polymerizable monomer that is a liquid. As used herein, a paste refers to a liquid in the form of a paste.

### <First Polymerizable Monomer>

The first polymerizable monomer that is a liquid is, for example, a liquid at normal temperature and pressure. The first polymerizable monomer is insoluble in a first solvent, which will be described later.

The first polymerizable monomer is not particularly limited, but is, for example, preferably a (meth)acrylate, and even more preferably a multifunctional (meth)acrylate having two or more (meth)acryloyloxy groups.

Examples of the first polymerizable monomer include ethoxylated bisphenol A dimethacrylate, neopentyl glycol dimethacrylate, urethane dimethacrylate, glycerol dimethacrylate, triethylene glycol dimethacrylate, tricyclodecane dimethanol dimethacrylate, and the like. The first polymerizable monomer may be used alone or in combination of two or more.

The content of the first polymerizable monomer in the dental composition is not particularly limited, but is, for example, preferably 1 mass% or more and 99.9 mass% or less, more preferably 1.5 mass% or more and 79 mass% or less, and even more preferably 2 mass% or more and 49 mass% or less.

When the content of the first polymerizable monomer in the dental composition is 1 mass% or more, the mechanical strength of the cured body of the dental composition is improved, and when the content is 99.9 mass% or less, the function or performance derived from the second polymerizable monomer of the cured body of the dental composition is improved.

The first component preferably further includes a hydrophobic filler.

Examples of the hydrophobic filler include a silane-treated glass filler and the like. The silane-treated glass filler is, for example, a barium glass treated with 3-methacryloyloxypropyltrimethoxysilane. The hydrophobic filler may be used alone or in combination of two or more.

The amount of the hydrophobic filler in the dental composition is preferably, for example, 45 to 75 mass%, more preferably 50 to 70 mass%, more preferably 55 to 65 mass%. When the amount of the hydrophobic filler in the dental composition is 45 mass% or more, the mechanical strength of the cured body of the dental composition is improved, and when the amount is 75 mass% or less, the dispersibility of the dental composition is improved, and the operability of the dental composition can be improved.

The first component may contain an inorganic filler other than the hydrophobic filler, as other components.

Examples of the inorganic filler include anhydrous silicate powder, fumed silica, alumina powder, glass powder (for example, barium glass powder, fluoroaluminosilicate glass powder), and the like. These inorganic fillers may be treated with a surface treatment agent such as a silane coupling agent and the like. The inorganic filler may be used alone or in combination of two or more.

The amount of the inorganic filler in the dental composition is, for example, preferably 0.01 mass% or more and 30 mass% or less, more preferably 0.05 mass% or more and 20 mass% or less, and even more preferably 0.1 mass% or more and 10 mass% or less.

When the amount of the inorganic filler in the dental composition is 0.01 or more and 30 mass% or less, the viscosity of the dental composition containing the glass is decreased, and the operability of the dental composition can be improved.

In the case where the hydrophobic filler and/or the inorganic filler are included, it is preferable to produce the first component in the form of a paste by using an automatic mortar for mixing. In the mixing of the first component in the form of a paste and the second component, because the filler has already been made into a paste, an automatic mortar is unnecessary, and a planetary centrifugal mixer or the like may be used.

### <Second Component>

The second component includes a first solvent in which the first polymerizable monomer is insoluble. As used herein, insoluble means that the amount of the first polymerizable monomer dissolved in 100 g of solvent at 25°C is less than 1 g.

### <First Solvent>

The first solvent is a solvent in which the first polymerizable monomer is insoluble. As used herein, the first solvent is an example of a solvent in which the first polymerizable monomer is insoluble in the method of producing the dental composition of the present embodiment.

The first solvent is not particularly limited to any solvent in which the first polymerizable monomer is insoluble, but is, for example, the first solvent may be water, glycerin, propylene glycol, ethylene glycol, polyethylene glycol having an average molecular weight of 1200 or less, butylene glycol, and the like. The first solvent may be used alone or in combination of two or more.

The content of the first solvent in the dental composition is not particularly limited, but is, for example, preferably 0.01 mass% or more and 40 mass% or less, more preferably 0.02 mass% or more and 35 mass% or less, and even more preferably 0.03 mass% or more and 30 mass% or less.

When the content of the first solvent in the dental composition is 0.01 mass% or more, the function or performance derived from the second polymerizable monomer of the cured body of the dental composition is improved, and when the content is 40 mass% or less, the light scattering and operability of the cured body of the dental composition are improved.

It is preferable that the second component further includes a second polymerizable monomer that is a solid.

### <Second Polymerizable Monomer>

The second polymerizable monomer that is a solid is a solid at normal temperature and pressure and is soluble in the first solvent described above. Soluble means that more than 10 g or more of the second polymerizable monomer is dissolved in 100 g of solvent at 25°C. The second polymerizable monomer is preferably insoluble in the first polymerizable monomer.

The second polymerizable monomer is preferably a (meth)acrylate, and more preferably a monofunctional (meth)acrylate having one (meth)acryloyloxy group.

The content of the second polymerizable monomer in the dental composition is not particularly limited, but is, for example, preferably 0.01 mass% or more and 99 mass% or less, more preferably 0.02 mass% or more and 79 mass% or less, and even more preferably 0.03 mass% or more and 49 mass% or less.

When the content of the second polymerizable monomer in the dental composition is 0.01 mass% or more, the function or performance derived from the second polymerizable monomer of the cured body of the dental composition is improved, and when the content is 99 mass% or less, the light scattering and operability of the cured body of the dental composition are improved.

When the first solvent is water, the second polymerizable monomer may be dissolved in water by allowing the second polymerizable monomer to absorb moisture in the atmosphere.

The content of the first solvent in the dental composition is not particularly limited but is preferably 0.01 mass% or more and 40 mass% or less, more preferably 0.02 mass% or more and 35 mass% or less, and even more preferably 0.03 mass% or more and 30 mass% or less.

When the content of the first solvent in the dental composition is 0.01 mass% or more, the function or performance derived from the second polymerizable monomer of the cured body of the dental composition is improved, and when the content is 40 mass% or less, the light scattering and operability of the cured body of the dental composition are improved.

The mass ratio of the first solvent to the second polymerizable monomer is preferably 0.01 or more and 5 or less, and even more preferably 0.1 or more and 2 or less. When the mass ratio of the first solvent to the second polymerizable monomer is 0.01 or more, the stability when the second polymerizable monomer is dissolved in the first solvent is improved, and when the mass ratio is 5 or less, the mechanical strength of the cured body of the dental composition is improved.

The mass ratio of the first polymerizable monomer to the total amount of the second polymerizable monomer and the first solvent is preferably 0.1 or more and 100 or less, and even more preferably 0.5 or more and 50 or less. When the mass ratio of the first polymerizable monomer to the total amount of the second polymerizable monomer and the first solvent is 0.1 or more, the mechanical strength of the cured body of the dental composition is improved, and when the mass ratio is 100 or less, the performance derived from the second polymerizable monomer of the cured body of the dental composition is improved.

In the method of producing the dental composition according to the present embodiment, the first component in the form of a paste including the first polymerizable monomer that is a liquid and the second component including the first solvent in which the first polymerizable monomer is insoluble are mixed, as described above. Accordingly, the dispersibility of the second component in the first component can be controlled. Thus, the second component can be uniformly localized in the first component.

As a result, in the method of producing the dental composition according to the present embodiment, when the dental composition is cured, the first component and the second component become in a state similar to a composite filler, and light scattering is obtained. Therefore, according to the present embodiment, the dental composition having excellent light scattering of the cured body is obtained.

In the method of producing the dental composition according to the present embodiment, even when the conventional organic-inorganic composite filler is not blended, the cured body with high light scattering can be obtained, as described above. Therefore, according to the present embodiment, it is possible to prevent deterioration in operability due to the addition of the conventional organic-inorganic composite filler.

In the present embodiment, by including the hydrophobic filler in the first component, the dispersibility of the first component that is a paste can be improved, as described above. In addition, by mixing the first component with the second component, the dispersibility of the dental composition can be improved. Further, by including the hydrophobic filler in the first component, the light scattering of the cured body can be improved without deteriorating the operability.

In the present embodiment, the second component includes the second polymerizable monomer that is a solid, as described above. Accordingly, when the first component is mixed with the second component, the first polymerizable monomer, the second polymerizable monomer, and the first solvent are mixed.

At this time, if the first polymerizable monomer and the second polymerizable monomer are mixed without using the first solvent, it is difficult to control the particle size of the second polymerizable monomer, and consequently the second polymerizable monomer cannot be uniformly localized in the first polymerizable monomer. As a result, the appearance and strength of the cured body of the dental composition deteriorates.

In addition, if the first polymerizable monomer and the second polymerizable monomer are mixed without using the first solvent, the second polymerizable monomer functions as a filler having a polarity different from that of the first polymerizable monomer. As a result, the viscosity of the dental composition increases significantly and the operability of the dental composition deteriorates.

In contrast, in the present embodiment, by mixing the first polymerizable monomer in the form of a paste that is insoluble in the first solvent, the second polymerizable monomer that is soluble in the first solvent, and the first solvent, the second polymerizable monomer can be uniformly localized in the dental composition. As a result, the dental composition can be obtained that is excellent in light scattering and hardly deteriorates in operability while imparting the function or performance derived from the second polymerizable monomer to the cured body of the dental composition.

Further, in the present embodiment, because the second polymerizable monomer that is soluble in the first solvent is included in the second component, the second polymerizable monomer can function as a filler having a polarity different from that of the first polymerizable monomer in the dental composition. Accordingly, in the present embodiment, with such a second polymerizable monomer, it is possible to prevent the dental composition from changing during storage and thereby deteriorating in the light scattering.

The second polymerizable monomer preferably includes a functional group that exhibits at least one of antibacterial and antiviral properties. The functional group that exhibits at least one of the antibacterial and antiviral properties means that one functional group exhibits both antibacterial and antiviral properties and that one functional group exhibits either antibacterial or antiviral properties. The functional group that exhibits antibacterial and antiviral properties also means that one functional group that exhibits an antibacterial property and another functional group that exhibits an antiviral property are included.

The functional group that exhibits at least one of the antibacterial and antiviral properties is not particularly limited, but is, for example, a quaternary ammonium base and the like. The quaternary ammonium base may function as the functional group that exhibits the antibacterial property (hereinafter referred to as an antibacterial group), and may also function as the functional group that exhibits the antiviral property (hereinafter referred to as an antiviral group) .

Examples of the second polymerizable monomer having the functional group that exhibits at least one of the antibacterial and antiviral properties includes monofunctional polymerizable monomers such as 2-(methacryloyloxy)ethyltrimethylammonium chloride, (3-acrylamidopropyl)trimethylammonium chloride, (2-(acryloyloxy)ethyl)trimethylammonium chloride, N-(2-acryloyloxyethyl)-N-benzyl-N,N-dimethylammonium chloride, dimethylaminopropylacrylamide methyl chloride quaternary salt, dimethylaminoethylacrylate methyl chloride quaternary salt, dimethylaminoethylacrylate benzyl chloride quaternary salt, and the like; polyfunctional polymerizable monomers such as diallyl-type quaternary ammonium salts such as diallyldimethylammonium chloride, diallyldiethylammonium chloride, and compounds represented by the following chemical formula; and the like.

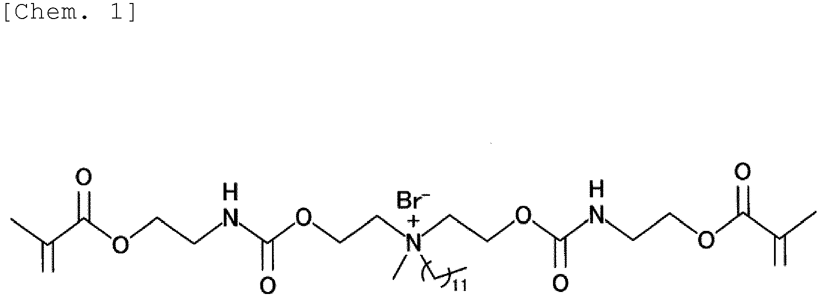

The second polymerizable monomer having the functional group that exhibits at least one of the antibacterial and antiviral properties may be used alone or in combination of two or more.

Examples of the second polymerizable monomer having neither the antibacterial group nor the antiviral group include phenoxyethylene glycol methacrylate, dimethylaminoethyl methacrylate, and the like.

The second polymerizable monomer having neither the antibacterial group nor the antiviral group may be used alone or in combination of two or more.

In the present embodiment, when the second polymerizable monomer has the antibacterial group, it is possible to impart the antibacterial property to the cured body of the dental composition, as the function or performance derived from the second polymerizable monomer. When the second polymerizable monomer has the antiviral group, it is possible to impart the antiviral property to the cured body of the dental composition, as the function or performance derived from the second polymerizable monomer.

In the present embodiment, a surfactant may or may not be added when the first polymerizable monomer, the second polymerizable monomer, and the first solvent are mixed.

The surfactant is not particularly limited as long as it is possible to improve the dispersibility of the solution, but is, for example, sodium lauryl sulfate, glycerin fatty acid esters, and the like. The surfactant may be used alone or in combination of two or more.

In the present embodiment, in the mixing step, a photopolymerization initiator, a tertiary amine, a polymerization inhibitor, and the like, are preferably added and kneaded, as other components.

Examples of the photopolymerization initiator include camphorquinone, phenylbis (2,4,6-trimethylbenzoyl) phosphine oxide, 2,4,6-trimethylbenzoyldiphenylphosphine oxide, benzyl ketal, diacetyl ketal, benzyl dimethyl ketal, benzyl diethyl ketal, benzyl bis(2-methoxyethyl) ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7 trifluoromethylthioxanthone, thioxanthone-10,10 dioxide, thioxanthone-10 oxide, benzoin methyl ether, benzoin ethyl ether, benzoin isopropyl ether, benzoin isobutyl ether, benzophenone, bis(4-dimethylaminophenyl) ketone, 4,4'-bis(diethylamino) benzophenone, and the like. The photopolymerization initiator may be used alone or in combination of two or more.

The tertiary amine may be either a tertiary aliphatic amine or a tertiary aromatic amine, but it is preferably the tertiary aromatic amine, and more preferably an alkyl p-dialkylaminobenzoate.

Examples of the tertiary aliphatic amine include N,N-dimethylaminoethyl methacrylate, triethanolamine, and the like.

Examples of the alkyl p-dialkylaminobenzoate include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, propyl p-diethylaminobenzoate, and the like.

Examples of the tertiary aromatic amine other than the alkyl p-dialkylaminobenzoate include 7-dimethylamino-4 methylcoumarin, N,N-dimethylaniline, N,N-dibenzylaniline, N,N-dimethyl-p-toluidine, N,N-diethyl-p-toluidine, N,N-bis(2-hydroxyethyl)-p-toluidine, N,N,2,4,6-pentamethylaniline, N,N,2,4-tetramethylaniline, N,N-diethyl-2,4,6-trimethylaniline, and the like.

The tertiary amine may be used alone or in combination of two or more.

Examples of the polymerization inhibitor include dibutylhydroxytoluene (2,6-di-tert-butyl-p-cresol), 6-tert-butyl-2,4-xylenol, and the like. The polymerization inhibitor may be used alone or in combination of two or more.

Other components may be added to the first polymerizable monomer, the second polymerizable monomer, and/or the first solvent, before mixing in the mixing step. Other components may be added after mixing the first polymerizable monomer, the second polymerizable monomer, and the first solvent, in the mixing step.

The mixing step may include a step of dissolving the second polymerizable monomer in the first solvent to obtain a solution, and a step of mixing the solution and the first component.

When the solution in which the first solvent has the second polymerizable monomer dissolved therein is mixed with the first component including the first polymerizable monomer, the second polymerizable monomer can be more uniformly localized in the dental composition. As a result, it is possible to further improve the light scattering and operability of the cured body of the dental composition, while imparting the function or performance derived from the second polymerizable monomer to the cured body of the dental composition.

For example, when the second polymerizable monomer has the antibacterial group, it is possible to impart the antibacterial property to the cured body of the dental composition. When the second polymerizable monomer has the antiviral group, it is possible to impart the antiviral property to the cured body of the dental composition.

Examples of the method of mixing the solution in which the first solvent has the second polymerizable monomer dissolved therein with the first component including the first polymerizable monomer include: a method of kneading the solution with the first component using a mortar; a method of mixing the solution with the first component using a magnetic stirrer; a method of mixing the solution and the first component using a stirrer having a stirring blade; and the like.

Other components may be added to the solution and/or to the first polymerizable monomer, before mixing. Other components may be added after mixing the solution and the first component.

The mixing step may include: a step of dissolving at least a part of the first polymerizable monomer and the second polymerizable monomer in a second solvent to obtain a solution; a step of removing the second solvent from the solution by distillation to obtain a dispersion in which at least a part of the first polymerizable monomer has the second polymerizable monomer dispersed therein; and a step of mixing the dispersion and the first solvent.

### <Second Solvent>

The second solvent is not particularly limited, but is, for example, an organic solvent such as ethanol, acetone, hexane, and the like. The second solvent may be used alone or in combination of two or more.

The content of the second solvent in the solution is not particularly limited, but is, for example, preferably 10 mass% or more and 99 mass% or less. When the content of the second solvent in the solution is 10 mass% or more, the dissolution stability of the polymerizable monomer is improved, and when the content is 99 mass% or less, the second solvent is easily removed by distillation from the solution.

In the present embodiment, when the dispersion in which the first polymerizable monomer has the second polymerizable monomer dispersed therein is mixed with the first solvent, the second polymerizable monomer can be more uniformly localized in the dental composition. As a result, the appearance and strength of the cured body of the dental composition can be further improved while imparting the function or performance derived from the second polymerizable monomer to the cured body of the dental composition.

For example, when the second polymerizable monomer has the antibacterial group, the antibacterial property can be imparted to the cured body of the dental composition. When the second polymerizable monomer has the antiviral group, the antiviral property can be imparted to the cured body of the dental composition.

When a part of the first polymerizable monomer is used in obtaining the solution, the dispersion is mixed with the remainder of the first polymerizable monomer after obtaining the dispersion. The timing of mixing the dispersion with the remainder of the first polymerizable monomer is not particularly limited, but it is preferable to mix the remainder of the first polymerizable monomer when mixing the dispersion with the first solvent.

The mass ratio of the second polymerizable monomer to at least a part of the first polymerizable monomer in obtaining the solution is preferably 0.01 or more and 100 or less, and more preferably 0.05 or more and 50 or less. When the mass ratio of the second polymerizable monomer to the first polymerizable monomer is 0.01 or more and 100 or less, the dispersion state of the dispersion obtained by removing the second solvent from the solution by distillation is favorable.

Examples of the method of mixing the dispersion and the first solvent include: a method of stirring the dispersion and the first solvent; a method of mixing the dispersion and the first solvent using a magnetic stirrer; a method of mixing the dispersion and the first solvent using a stirrer having a stirring blade; and the like.

In the present embodiment, a surfactant may or may not be added when the dispersion and the first solvent are mixed.

The surfactant is not particularly limited as long as it is possible to improve the dispersibility of the solution, but is, for example, sodium lauryl sulfate, glycerin fatty acid ester, and the like. The surfactant may be used alone or in combination of two or more.

In the present embodiment, after mixing the dispersion and the first solvent, it is preferable to add and knead the hydrophobic filler, inorganic filler, photopolymerization initiator, tertiary amine, polymerization inhibitor, and the like, as described above, as other components.

Other components may be added to the dispersion and/or to the first solvent, before mixing. Other components may be added when the dispersion and the first solvent are mixed.

### <Dental Composition>

In the dental composition according to the present embodiment, the first component has the second component dispersed therein. Here, the first component is a paste including the first polymerizable monomer that is a liquid, and the second component includes the solvent in which the first polymerizable monomer is insoluble.

The dental composition according to the present embodiment can be produced by the method of producing the dental composition described above. As the first component used in the dental composition according to the present embodiment, the first component used in the method of producing the dental composition described above can be used. As the first polymerizable monomer, the first polymerizable monomer used in the method of producing the dental composition described above can be used.

As the second component used in the dental composition according to the present embodiment, the second component used in the method of producing the dental composition described above can be used. As the solvent used in the dental composition according to the present embodiment, the first solvent used in the method of producing the dental composition described above can be used.

The content of the first polymerizable monomer in the dental composition is not particularly limited, but is, for example, preferably 1 mass% or more and 99.9 mass% or less, more preferably 1.5 mass% or more and 79 mass% or less, and even more preferably 2 mass% or more and 49 mass% or less.

The content of the first solvent in the dental composition is not particularly limited, but is, for example, preferably 0.01 mass% or more and 40 mass% or less, more preferably 0.02 mass% or more and 35 mass% or less, and even more preferably 0.03 mass% or more and 30 mass% or less.

In the dental composition according to the present embodiment, the first component in the form of a paste including the first polymerizable monomer that is a liquid and the second component including the first solvent in which the first polymerizable monomer is insoluble are included. Accordingly, the same effect as the dental composition obtained by the production method described above can be obtained. Specifically, in the dental composition according to the present embodiment, the dispersibility of the second component in the first component is controlled, and the second component can be uniformly localized in the first component.

Therefore, in the present embodiment, when the dental composition is cured, the first component and the second component become in a state similar to a composite filler, and light scattering is obtained. Therefore, the dental composition according to the present embodiment can be excellent in light scattering of the cured body.

In the present embodiment, even when the conventional organic-inorganic composite filler or the like is not blended, the cured body with high light scattering can be obtained, as described above. Therefore, in the dental composition according to the present embodiment, it is possible to prevent deterioration in operability due to the addition of the conventional organic-inorganic composite filler.

In the dental composition according to the present embodiment, it is preferable that the second component further includes the second polymerizable monomer that is a solid. It is also preferable that the second polymerizable monomer is soluble in the first solvent described above. As the second polymerizable monomer, the second polymerizable monomer used in the method of producing the dental composition described above can be used.

The content of the second polymerizable monomer in the dental composition is not particularly limited, but is, for example, preferably 0.01 mass% or more and 99 mass% or less, more preferably 0.02 mass% or more and 79 mass% or less, and even more preferably 0.03 mass% or more and 49 mass% or less.

In the dental composition according to the present embodiment, by including such a second polymerizable monomer in the second component, the same effect as the dental composition obtained by the production method described above can be obtained. Specifically, in the dental composition according to the present embodiment, by mixing the first polymerizable monomer in the form of a paste that is insoluble in the first solvent, the second polymerizable monomer that is soluble in the first solvent, and the first solvent, the second polymerizable monomer can be uniformly localized in the dental composition.

Therefore, the dental composition according to the present embodiment can be a dental composition in which the function or performance derived from the second polymerizable monomer is imparted to the cured body of the dental composition, and which is excellent in light scattering and hardly deteriorates in operability.

In the dental composition according to the present embodiment, the second polymerizable monomer preferably includes the functional group that exhibits at least one of the antibacterial and antiviral properties. As the second polymerizable monomer having the functional group that exhibits at least one of the antibacterial and antiviral properties, the second polymerizable monomer having the antibacterial and/or antiviral groups used in the method of producing the dental composition described above can be used.

Specifically, as the antibacterial and/or antiviral groups used in the second polymerizable monomer, a quaternary ammonium base and the like used in the method of producing the dental composition described above can be used.

In the dental composition according to the present embodiment, because the second polymerizable monomer has the antibacterial and/or antiviral groups, when the second polymerizable monomer has the antibacterial group, the antibacterial property can be imparted to the cured body of the dental composition as the function or performance derived from the second polymerizable monomer. When the second polymerizable monomer has the antiviral group, the antiviral property can be imparted to the cured body of the dental composition as the function or performance derived from the second polymerizable monomer.

The use of the dental composition according to the present embodiment is not particularly limited, but is, for example, the dental composition according to the present embodiment can be used for a dental composite resin, a dental cement, a denture base resin, a dental general-purpose resin, and the like.

The dental composition according to the present embodiment can be the dental composition that is excellent in light scattering and hardly deteriorates in operability, as described above. Among these, the dental composition according to the present embodiment can be preferably used for the dental composite resin, and more preferably used for a flowable composite resin. In the case where the dental composition according to the present embodiment is used for the flowable composite resin, the flowable composite resin may be provided as a one-part formulation or as a two-part formulation.

In the case where the dental composition according to the present embodiment is the flowable composite resin, extrusion hardness of the flowable composite resin may be typically set to 10 kgf or less. Accordingly, the formability and operability of the flowable composite resin can be improved.

The manner of providing the flowable composite resin is not particularly limited, but is, for example, it is provided as a package including a syringe filled with the flowable composite resin, a plunger fitted into the syringe from the rear end side of the syringe, and a needle tip attached to the tip of the syringe.

In the case where the flowable composite resin is provided with such a needle tip, the inner diameter of the needle of the needle tip may be 0.3 mm or more and 0.9 mm or less.

In the case where the flowable composite resin is provided as the two-part formulation, the package may include, for example, two syringes connected in parallel and two plungers connected in parallel, with a static mixer at the tips of the syringes.

The dental composition according to the present embodiment is suitably used for products in the field of dentistry in which performance such as antibacterial and antiviral properties is required, from the viewpoint that the antibacterial and/or antiviral properties can be imparted to the cured body of the dental composition as the function or performance derived from the second polymerizable monomer, as described above.

### Example

Hereinafter, the present embodiment will be further described with reference to examples. In the following, numerical values without units or "%" are based on mass (mass%) unless otherwise specified.

### <Preparation of Original Liquid>

An original liquid was prepared as follows. As a polymerizable monomer component, 120 g of bisphenol A polyethoxymethacrylate (BPE-80N), 30 g of neopentylglycol dimethacrylate (NPG), and 30 g of tricyclodecane dimethanol dimethacrylate (DCP) were weighed, and then 1.5 g of camphorquinone (CQ), 3.0 g of ethyl p-dimethylaminobenzoate (EPA), and 1.2 g of 6-tert-dibutyl-2,4-xylenol (BHT) were added and mixed uniformly using a stirrer.

### <Preparation of Paste>

3.5 g of the prepared original liquid was weighed, 0.16 g of AEROSIL R812 (fine silica particles surface-treated with hexamethyldisilazane, manufactured by Nippon Aerosil Co., Ltd., AEROSIL (registered trademark) R812) was weighed and added, and the mixture was kneaded using an automatic mortar. After the mixture was kneaded for 5 minutes, 8 g of a silane-treated glass filler was weighed and added in eight separate batches, and the mixture was kneaded. After the silane-treated glass filler was added, the mixture was kneaded so that the total kneading time was 30 minutes, and a uniform paste was obtained.

### <Addition of Later Component>

7 g of the prepared paste was weighed, and later additive components were added as presented in Table 1 below and blended with a spatula. Then, the mixture was kneaded at 800 rpm for 3 minutes using a planetary centrifugal mixer, and a flowable composite resin was obtained.

**[Table 1]**

| | | EXAMPLE 1 | EXAMPLE 2 | EXAMPLE 3 | EXAMPLE 4 | EXAMPLE 5 | EXAMPLE 6 | COMPARATIVE EXAMPLE 1 | COMPARATIVE EXAMPLE 2 |
|---|---|---|---|---|---|---|---|---|---|
| | NON-COLORED PASTE | 7 | 7 | 7 | 7 | 7 | 7.686 | 7 | 7 |
| LATER ADDITIVE COMPONENT | MTMAC AQUEOUS SOLUTION (WATER 19.3%) | 0.45 | - | 0.85 | - | - | - | - | - |
| | DADMAC AQUEOUS SOLUTION (WATER 35%) | - | 0.45 | - | 0.85 | - | - | - | - |
| | UDMA | - | - | - | - | 0.45 | - | 0.85 | - |
| | NPG | 0.23 | 0.23 | - | - | 0.23 | - | - | ₋ |
| | WATER | 0.17 | 0.17 | - | - | 0.17 | 0.17 | - | - |
| | ORGANIC FILLER | - | - | - | - | - | - | - | 0.85 |
| | TOTAL | 7.85 | 7.85 | 7.85 | 7.85 | 7.85 | 7.856 | 7.85 | 7.85 |

The preparation of the original liquid and the production of the paste were performed in the same manner in all Examples and Comparative Examples. The composition of the formulation was changed in the addition of the later component (s). Table 2 presents the composition of the formulation of Examples and Comparative Examples.

**[Table 2]**

| | | | EXAMPLE 1 | EXAMPLE 2 | EXAMPLE 3 | EXAMPLE 4 | EXAMPLE 5 | EXAMPLE 6 | COMPARATIVE EXAMPLE 1 | COMPARATIVE EXAMPLE 2 |
|---|---|---|---|---|---|---|---|---|---|---|
| ORIGINAL LIQUID COMPONENT | NON-COLORED COMPONENT | BPE-80N | 17.14 | 17.14 | 17.14 | 17.14 | 17.14 | 18.8 | 17.14 | 17.14 |
| | | NPG | 4.29 | 4.29 | 4.29 | 4.29 | 4.29 | 4.71 | 4.29 | 4.29 |
| | | DCP | 4.29 | 4.29 | 4.29 | 4.29 | 4.29 | 4.71 | 4.29 | 4.29 |
| | | CQ | 0.21 | 0.21 | 0.21 | 0.21 | 0.21 | 0.23 | 0.21 | 0.21 |
| | | EPA | 0.43 | 0.43 | 0.43 | 0.43 | 0.43 | 0.47 | 0.43 | 0.43 |
| | | BHT | 0.17 | 0.17 | 0.17 | 0.17 | 0.17 | 0.19 | 0.17 | 0.17 |
| | | AEROSIL R812 | 1.21 | 1.21 | 1.21 | 1.21 | 1.21 | 1.33 | 1.21 | 1.21 |
| | | SILANE-TREATED GLASS FILLER | 61.43 | 61.43 | 61.43 | 61.43 | 61.43 | 67.4 | 61.43 | 61.43 |
| LATER ADDITIVE COMPONENT | | MTMAC AQUEOUS SOLUTION (WATER 19.3%) | 5.73 | - | 10.83 | - | - | - | - | - |
| | | DADMAC AQUEOUS SOLUTION (WATER 35%) | - | 5.73 | - | 10.83 | - | - | - | - |
| | | UDMA | - | - | - | -- | 5.73 | - | 10.83 | - |
| | | NPG | 2.93 | 2.93 | - | - | 2.93 | - | - | - |
| | | WATER | 2.17 | 2.17 | - | - | 2.17 | 2.16 | - | - |
| | | ORGANIC FILLER | - | - | - | - | - | - | - | 10.83 |
| | | TOTAL | 100 | 100 | 100 | 100 | 100 | 100 | 100 | 100 |

In Tables 1 and 2, abbreviations have the following meanings.
BPE-80N: bisphenol A polyethoxymethacrylate
NPG: neopentylglycol dimethacrylate
DCP: tricyclodecane dimethanol dimethacrylate
CQ: camphorquinone
EPA: ethyl p-dimethylaminobenzoate
BHT: 6-tert-dibutyl-2,4-xylenol
MTMAC: 2-(methacryloyloxy)ethyltrimethylammonium chloride
DADMAC: diallyldimethylammonium chloride
UDMA: di-2-methacryloyloxyethyl-2,2,4-trimethylhexamethylenedicarbamate

The refractive indices of components contained in the original liquid are 1.543 for BPE-80N, 1.452 for NPG, 1.500 for DCP, 1.460 for MTMAC, 1.480 for DADMAC, 1.333 for water, 1.53 for barium glass (manufactured by Schott AG, G018-053), and 1.457 for silicon dioxide (SiO₂).

In Table 2, the silane-treated glass filler is barium glass powder (manufactured by Schott AG, G018-053 UF, 0.4 µm) obtained by silane treatment with 3-methacryloyloxypropyltrimethoxysilane having a weight ratio of 8 w/w% to glass. The silane treatment was carried out with reference to PTL 2 (Patent No. 6793241) of Citation List.

In Table 2, the organic filler is an organic-inorganic composite filler prepared by referring to Japanese Laid-Open Patent Publication No. 2011-68596. Specifically, the organic-inorganic composite filler is obtained as follows. Glass powder including Si₂O, SrO, B₂O₃, BaO, Al₂O₃, Na₂O, CaO, ZnO, La₂O₃, WO₃, ZrO₂, TiO₂, Nb₂O₅, F, and the like, as main components, was mixed with a (meth)acrylate compound. The mixture was polymerized and cured, and the resulting cured body was pulverized.

The light scattering property, transparency, paste fluidity, and aesthetics of Examples and Comparative Examples were evaluated as follows. The evaluation results are presented in Table 3.

### <Light Scattering Property>

The obtained paste of the flowable composite resin was applied evenly to the surface of an acrylic plate to prepare a disk-shaped test piece with a diameter of 15 mm and a thickness of 1 mm (FIGS. 1 and 2). Using a light irradiator (G-Light Prima II Plus, manufactured by GC Corporation), both the front and back sides of the test piece were irradiated for 10 seconds nine times each to prepare a cured body, which was used as a test specimen (FIG. 3). The light scattering property (light scattering) was evaluated using a goniophotometer (manufactured by Murakami Color Research Laboratory Co., Ltd.) under a measurement range of ±90. The evaluation criteria were as follows.
Good: The measurement angle at which the transmitted light intensity falls to or below 10% for the first time is outside the range of ±30.
Poor: The measurement angle at which the transmitted light intensity falls to or below 10% for the first time is within the range of ±30.

### <Transparency>

The test specimens obtained in the light scattering property test described above (FIG. 3) were evaluated for transparency by measuring the total light transmittance (T.T.) using a haze meter (manufactured by Nippon Denshoku Industries Co., Ltd.). Transparency was evaluated as good (transparent) when the total light transmittance (T.T.) was 35% or higher.

### <Paste Fluidity>

The obtained paste of the flowable composite resin was applied to the surface of an acrylic plate under the same conditions as the light scattering property test described above (FIGS. 1 and 2), and its appearance was examined and its fluidity was evaluated. The evaluation criteria were as follows.
Good: flowable appearance (with fluidity)
Poor: putty appearance (without fluidity)

### <Aesthetics>

The test specimens obtained in the light scattering property test (FIG. 3) were evaluated for appearance. In the evaluation of appearance, visually observable irregularities and obvious cloudiness were examined on the prepared cured bodies (FIG. 3). The evaluation criteria for aesthetics were as follows. Even when the evaluation of aesthetics was poor, the dental composition was evaluated as good when the evaluation of transparency described above was good.
Good: without irregularities and without cloudiness
Poor: with irregularities and/or cloudiness

**[Table 3]**

| | EXAMPLE 1 | EXAMPLE 2 | EXAMPLE 3 | EXAMPLE 4 | EXAMPLE 5 | EXAMPLE 6 | COMPARATIVE EXAMPLE 1 | COMPARATIVE EXAMPLE 2 |
|---|---|---|---|---|---|---|---|---|
| LATER ADDITIVE COMPONENT | MTMAC | DADMAC | MTMAC | DADMAC | UDMA | - | UDMA | ORGANIC FILLER |
| LIGHT SCATTERING | GOOD | GOOD | GOOD | GOOD | GOOD | GOOD | POOR | GOOD |
| TRANSPARENCY [%] | 53.83 | 58.99 | 58.15 | 63.39 | 40.61 | 49.23 | 82.53 | |
| PASTE FLUIDITY | GOOD | GOOD | GOOD | GOOD | GOOD | GOOD | GOOD | POOR |
| AESTHETICS | GOOD | GOOD | GOOD | POOR | POOR | POOR | GOOD | GOOD |

From Table 3, Examples 1 to 6 were favorable in light scattering property. transparency. amd paste fluidity. Among these. Examples 1 to 3 were also favorable in aesthetics.

In contrast, comparative Example 1 was poor in light scattering, and Comparative Example 2 was poor in paste fluidity.

In Example 1, it is considered that, in a flowable composite resin 20 fixed to an acrylic plate (object) 10 before curing, a solution 40 in which the first solvent has the second polymerizable monomer dissolved therein is uniformly dispersed in a first polymerizable monomer 30 (the droplet diameter of the solution 40 in which the first solvent has the second polymerizable monomer dissolved therein is small) (see FIGS. 1 and 2).

Although the embodiments of the present invention have been described above, the present invention is not limited to specific embodiments, and various modifications and changes are possible within the scope of the invention described in the claims.

The present application claims priority to Japanese Patent Application No. 2021-62369, filed March 31, 2021, with the Japanese Patent Office, the contents of which are incorporated herein by reference in their entirety.

### Description of the Reference Numeral

- 10: acrylic plate (object)
- 20: flowable composite resin
- 30: first polymerizable monomer
- 40: solution in which first solvent has second polymerizable monomer dissolved therein

## Claims

1. A method of producing a dental composition in which a first component has a second component dispersed therein, wherein
the first component is a paste including a first polymerizable monomer that is a liquid,
the second component includes a solvent in which the first polymerizable monomer is insoluble, and
the method includes a step of mixing the first component and the second component.

2. The method of producing the dental composition according to claim 1, wherein the first component further includes a hydrophobic filler.

3. The method of producing the dental composition according to claim 1 or 2, wherein
the second component further includes a second polymerizable monomer that is a solid, and
the second polymerizable monomer is soluble in the solvent.

4. The method of producing the dental composition according to claim 3, wherein the step of mixing includes:
a step of dissolving the second polymerizable monomer in the solvent to obtain a solution; and
a step of mixing the solution and the first component.

5. The method of producing the dental composition according to any one of claims 1 to 4, wherein the dental composition is a flowable composite resin.

6. A dental composition obtained by the method of producing the dental composition of any one of claims 1 to 5.

7. A dental composition in which a first component has a second component dispersed therein, wherein
the first component is a paste including a first polymerizable monomer that is a liquid, and
the second component includes a solvent in which the first polymerizable monomer is insoluble.

8. The dental composition according to claim 7,
wherein the second component further includes a second polymerizable monomer that is a solid, and
wherein the second polymerizable monomer is soluble in the solvent.
